# EUROPEAN PATENT APPLICATION

(11) **EP 2 524 714 A1**
(43) Date of publication of application: **21.11.2012**
(21) Application number: 11732815.3
(22) Date of filing: 14.01.2011
(51) Int. Cl.: A61M 16/10, C01B 13/02, B01D 53/04

(54) **OXYGEN CONCENTRATION DEVICE**

(30) Priority: 15.01.2010 JP 2010007199
(71) Applicant: Terumo Kabushiki Kaisha, Tokyo 151-0072 (JP); Ikiken Co., Ltd., Saitama 350-1331 (JP)
(72) Inventor: SUGAWARA, Kimio, Sayama-shi Saitama 350-1331 (JP); ENOMOTO, Hisashi, Sayama-shi Saitama 350-1331 (JP)
(74) Representative: TBK
(86) International application number: PCT/JP2011/000181
(87) International publication number: WO 2011/086943

(57) **Abstract**

To provide an oxygen concentrator that can ensure safety by interrupting oxygen in a case where the oxygen concentrator is exposed to fire or an abnormal high-temperature environment when a user thereof is inhaling oxygen with a cannula.

The oxygen concentrator includes: a compressor 10 separating compressed air by compressing raw air; an oxygen outlet 100 for discharging oxygen acquired from the compressed air; a coupler 17 mounted on a tube 72 of a cannula 70 and removably connecting the tube 72 to the oxygen outlet 100, and moreover having a temperature sensor 400; and a control unit 200 stopping operation of the compressor 10 to interrupt the supply of oxygen when a temperature sensed by the temperature sensor 400 reaches a predetermined temperature or higher.

## Description

### Technical Field

The present invention relates to an oxygen concentrator, and more particularly, to an oxygen concentrator that prevents fire spreading to the oxygen concentrator or fire in this device itself when being exposed to an abnormal high-temperature environment such as the fire, and the like.

### Background Art

An oxygen concentrator using pressure swing adsorption is configured to acquire concentrated oxygen by using zeolite selectively adsorbing nitrogen by transmitting oxygen in raw air as an adsorbent.
According to the oxygen concentrator using the method, introduced raw air is compressed by a compressor to generate compressed air and the compressed air is supplied to an adsorption column containing the adsorbent to separate oxygen by adsorbing nitrogen to the adsorbent. While the separated concentrated oxygen is stored in a tank, a predetermined flow of oxygen can be supplied from the tank through a pressure reducing valve or a flow setter to allow a patient to inhale oxygen by using a mechanism such as a nasal cannula, and the like.

When the oxygen concentrator is installed at a place where an AC power supply (utility AC power supply) can be used, for example, a domiciliary oxygen therapy patient having a deteriorated lung function can safely inhale oxygen even while sleeping to have a good sleep.

The oxygen concentrator used for a long-term oxygen inhalation therapy which is effective as a therapeutic method for a patient who suffers from respiratory disease, such as chronic bronchitis and the like, is generally not transportable and is not configured for the patient to take with them to go outside.
Meanwhile, an oxygen concentrator has also been conventional, which is configured to be small and portable so as for a patient to conveniently move out the door or move among rooms in a house or a medical facility or so as to be suitable for a limited placement space in home placement (see Patent Literature 1). An oxygen concentrator in which a temperature sensor is installed at a nasal cannula, and when the temperature reaches 50°C, separation of the concentrated oxygen stops is also proposed (see Patent Literature 2).

### Citation List

### Patent Literature

Patent Literature 1: Japanese Patent Application Laid-Open No. 2005-111016
Patent Literature 2: Japanese Patent Application Laid-Open No. 2009-183544

### Summary of Invention

### Technical Problem

However, in an oxygen concentrator of Patent Literature 1, a user connects a nasal cannula to an oxygen outlet of the oxygen concentrator through a tube and a coupler and inhales concentrated oxygen discharged from the oxygen outlet. However, when the user inhales the concentrated oxygen by using the nasal cannula, oxygen is supporting gas, and thus a smoking act or a fire using act around the oxygen concentrator is strictly prohibited. Nevertheless, for example, as the user smokes, an accident caused by ignition directly in the tube such as the nasal cannula, and the like may occur and in some cases, the fire may be expanded due to ignition of the oxygen concentrator itself.
A device of Patent Literature 2 has a configuration in which a temperature sensor is placed in a midstream of the nasal cannula and oxygen supplying stops by a sensing signal, but in a very simple structure disclosed in Patent Literature 2 in which oxygen supplying is stopped at approximately 50°C, for example, a heater is provided in a room equipped with the device and oxygen supplying is interrupted as radiant heat of the heater reaches the device or when an environmental temperature around the device in a summer closed room, and the like rises, the device may not be operated due to the temperature rise, and as a result, the device is inconvenient to use and low in operability.
Accordingly, an object of the present invention is to provide an oxygen concentrator that can certainly sense a high-temperature environment and ensure safety when the oxygen concentrator is exposed to fire or an abnormal high-temperature environment at the time of user inhaling oxygen with a cannula.

### Solution to Problem

An oxygen concentrator according to the present invention includes: concentrated oxygen separating means for separating concentrated oxygen from raw air; oxygen outlet for supplying the concentrated oxygen separated by the concentrated oxygen separating means; a coupler mounted on a tube of a cannula and removably connecting the tube to the oxygen outlet, and moreover having a temperature sensor; and a control unit stopping operation of the concentrated oxygen separating means to interrupt the supply of oxygen when a temperature sensed by the temperature sensor reaches a predetermined temperature or higher.
According to the configuration, safety may be ensured by interrupting oxygen when the oxygen concentrator is exposed to fire or an abnormal high-temperature environment at the time of user inhaling oxygen with the cannula.

In the oxygen concentrator of the present invention, the control unit stops operation of a compressor, which serves as the concentrated oxygen separating means, to interrupt the supply of oxygen when the temperature sensed by the temperature sensor reaches the predetermined temperature or higher and when a continued time, during which the temperature rise rate which is a temperature rise value per unit time reaches a predetermined value or more, is continued for a predetermined continued time or longer.
According to the configuration, the supply of oxygen may be certainly interrupted by sensing the rise in temperature of the oxygen outlet.

In the oxygen concentrator of the present invention, in the case where the temperature sensed by the temperature sensor reaches the predetermined temperature or higher and when the temperature rise rate is less than the predetermined value, the control unit stops the operation of the compressor to interrupt the supply of oxygen when the temperature sensed by the temperature sensor reaches an additionally set temperature or higher over the predetermined temperature.
According to the configuration, the supply of oxygen may be certainly interrupted by sensing the rise in temperature of the oxygen outlet and a main body of the oxygen concentrator may be prevented from being combusted in a spreading fire.

In the oxygen concentrator of the present invention, the temperature sensor may be a thermistor and the temperature sensor may be built in the oxygen outlet.
According to the configuration, since cost-down is achieved by using the thermistor which is cheap as the temperature sensor and further, the temperature sensor is built in the oxygen outlet, the temperature may be prevented from not being accurately sensed due to attachment such as a scale, and the like attached to the temperature sensor and the temperature may be prevented from not being accurately sensed due to moisture attached to the temperature sensor even when using a humidifier.

In the oxygen concentrator of the present invention, the coupler is made of the flame retardant resin material.
According to the configuration, flame is removed in the coupler when the supply of oxygen from the oxygen outlet stops.
In the oxygen concentrator of the present invention, the oxygen outlet is made of metal having thermal conductivity.
According to the configuration, heat by combustion of the coupler may be rapidly transferred to the temperature sensor through the oxygen outlet, and as a result, a temperature sensing time is shortened.
In the oxygen concentrator of the present invention, the oxygen concentrator includes warning means for notifying that oxygen supply from the oxygen outlet is interrupted by the command of the control unit and
a speaker notifying that oxygen supply from the oxygen outlet is interrupted of by the command of the control unit by voice.
According to the configuration, the oxygen concentrator may certainly notify the user of stopping the supply of oxygen.

### Advantageous Effects of Invention

The present invention can provide an oxygen concentrator that can ensure safety by interrupting oxygen when the oxygen concentrator is exposed to fire or an abnormal high-temperature environment at the time of user inhaling oxygen with a cannula.

### Brief Description of Drawings

FIG. 1 is a perspective view viewed from the front side, which illustrates an exterior of an embodiment of an oxygen concentrator of the present invention.
FIG. 2 is a bottom view of the exterior of the oxygen concentrator of FIG. 1.
FIG. 3 is a perspective view diagonally viewed from the rear side, which illustrates an internal structure example of the oxygen concentrator illustrated in FIGS. 1 and 2.
FIG. 4 is a diagram illustrating a compressor, a first connection conduit and a second connection conduit connected to the compressor, and a noise buffer also serving as an intake filter.
FIG. 5 is a perspective view illustrating a state in which a nasal cannula is connected to an oxygen outlet of an oxygen concentrator.
FIG. 6 is an exploded perspective view illustrating a configuration example of the nasal cannula.
FIG. 7 is a perspective view illustrating a coupler socket of the nasal cannula and the oxygen outlet.
FIG. 8 is an exploded perspective view illustrating a connector of a tube and the coupler socket of the nasal cannula and the oxygen outlet.
FIG. 9 is a cross-sectional view illustrating the vicinities of the oxygen outlet and the coupler socket.
FIG. 10 is a diagram illustrating a system configuration example of the oxygen concentrator.
FIG. 11 is a diagram illustrating an example of a change in temperature at the oxygen outlet until the concentrated oxygen stops to be extinguished from the time when the nasal cannula is ignited.
FIG. 12 is a flowchart illustrating an example of sensing the temperature in the oxygen concentrator of the present invention.

### Description of Embodiments

Hereinafter, an exemplary embodiment of the present invention will be described in detail with reference to the accompanying drawings.

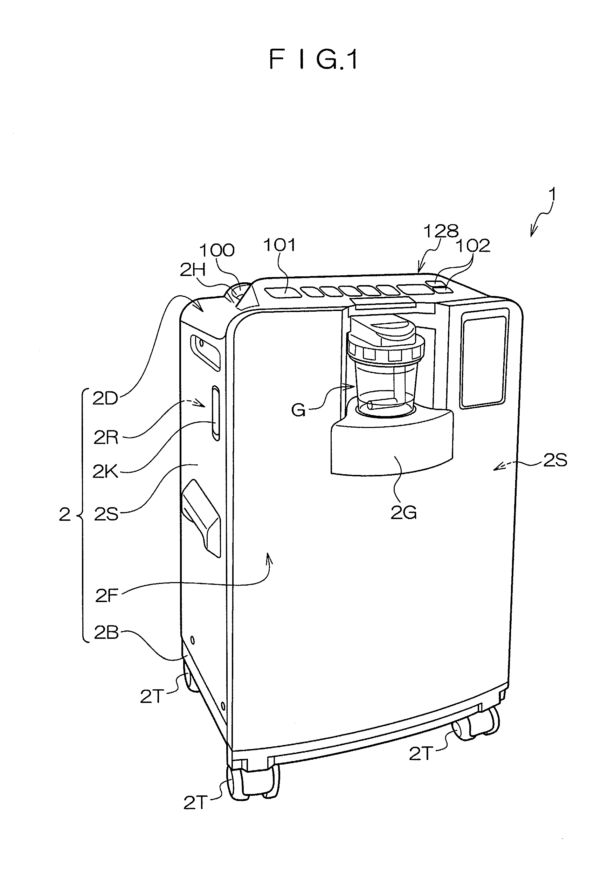

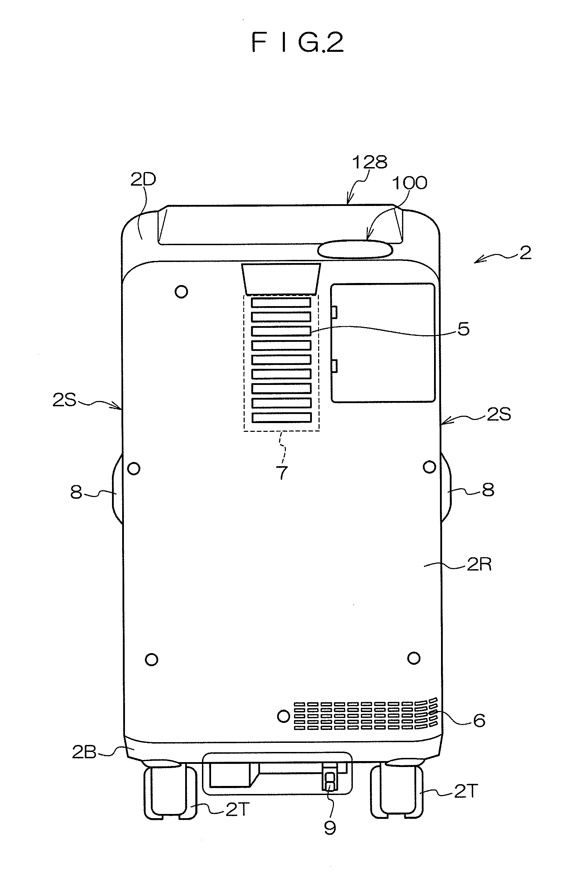

FIG. 1 is a front perspective view illustrating an exterior of an embodiment of an oxygen concentrator with a compressor of the present invention. FIG. 2 is a bottom view of the exterior of the oxygen concentrator of FIG. 1.
The oxygen concentrator 1 illustrated in FIGS. 1 and 2 is preferably a portable (also called transportable or movable) oxygen concentrator. The oxygen concentrator 1 illustrated in FIG. 1 uses for example, compressed air pressure swing adsorption (PSA) by compressed air as an oxygen separation principle.
In static pressure swing adsorption using only compressed air, only the compressed air is sent into an adsorption column body to adsorb nitrogen. The static swing adsorption has a merit in achieving miniaturization and light-weight of a compressor as compared with positive and negative swing adsorption (VPSA) by the compressed air and depressurized air.

An oxygen concentrator 50 illustrated in FIGS. 1 and 2 is, for example, an oxygen concentrator (a height of 62.5 cm, a width of 35 cm, a depth of 29.5 cm, and a weight of 23 kg) having the maximum supply flow of oxygen concentrated at 90% or more of 5 L class (5 L/min.), in which a setting unit of the oxygen flow may be set in the range of, for example, 0.25 L to 5.00 L (every 0.25 L in the range of 0.25 L to 2.00 L and every 0.50 L in the range of 2.00 L to 5.00 L). The oxygen concentrator 1 includes a substantially rectangular parallelepiped main case 2, a display unit 128 capable of setting a flow, a humidifier G, a cannular rack 2K, and casters 2T positioned at four edges.
The main case 2 includes a front panel 2F, left and right side panels 2S, and a rear panel 2R, a top 2D, and a bottom 2B. As illustrated in FIG. 1, a display unit 128 installed to be inclined at an angle of approximately 5 to 10° obliquely forward and upward, an oxygen outlet 100 installed at a concave portion 2H in the rear of the display unit 128 on the top 2D, a power switch 101 and an oxygen flow setting button 102 are placed on the top 2D. An upper part of the front panel 2F at substantuially the center thereof in the width direction is opened to form a placement section 2G to install therein the humidifier G. The casters 2T are placed at four edges of the bottom 2B and the oxygen concentrator 1 is movable by using the casters 2.

Referring to FIG. 2, in the rear panel 2R, an air introduction port 5 for introducing outdoor air into the main case 2 is formed at a central position of an upper part of the rear panel 2R and an exhaust port 6 for discharging warmed air in the main case 2 to the outside is formed at a lower right part of the rear panel 2R. An air introduction port filter 7 is removably mounted on an inner surface of the air introduction port 5. In addition, the left and right side panels 2S have handles 8 and the bottom 2B has a retractable power cord 9.

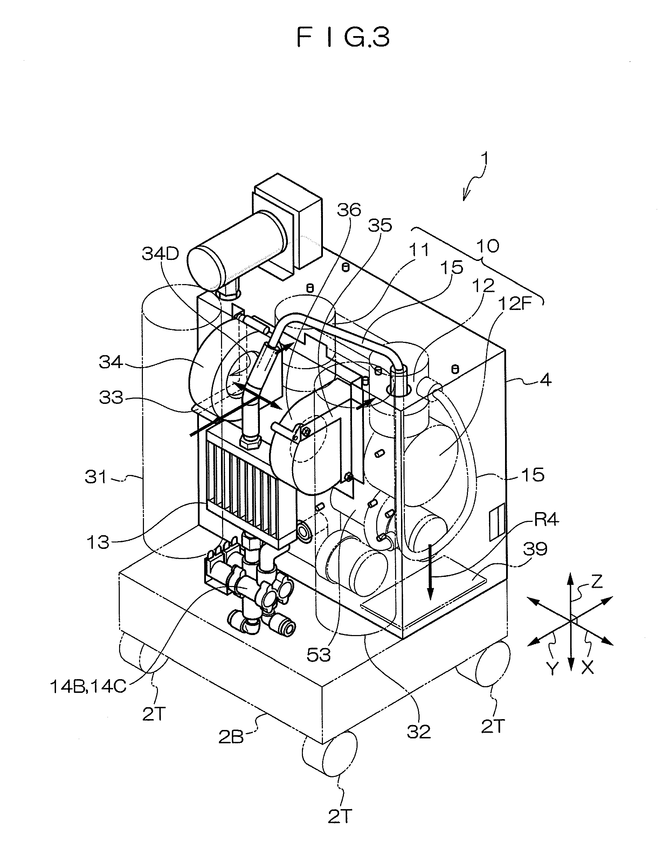

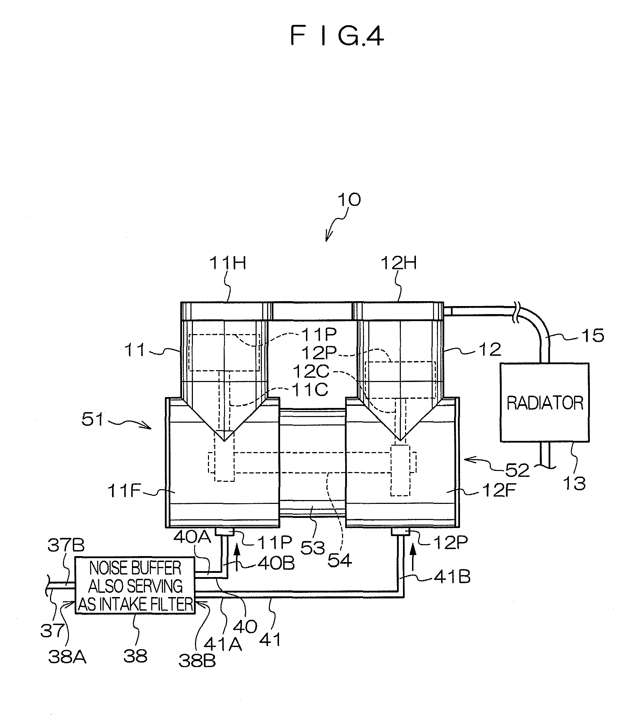

FIG. 3 is a perspective view illustrating an internal structure example of the oxygen concentrator 1 illustrated in FIGS. 1 and 2, which is diagonally viewed from the rear side. FIG. 4 is a diagram illustrating a compressor 10 constituting concentrated oxygen separating means for separating concentrated oxygen from raw air, a first connection conduit 40 and a second connection conduit 41 connected to the compressor 10, and a noise buffer 38 also serving as an intake filter.
As illustrated in FIG. 3, the compressor 10 is set on the bottom 2B and the compressor 10 is placed in a rectangular parallelepiped compressor case 4 for sound proofing. By using the non-woven fabric, a light weight and a soundproof effect are achieved. On a bottom surface of the compressor case 4, a first adsorption column body 31 and a second adsorption column body 32, which constitute the concentrated oxygen separating means for separating concentrated oxygen from raw air, are fixed while standing at an interval in an X direction (a horizontal direction) and in parallel in a Z direction (vertical direction).

As illustrated in FIG. 3, a sleeve 12 of the compressor 10 is connected to a conduit 15 and a cooling radiator 13 and 3-way switching valves 14B and 14C are connected to the midstream of the conduit 15. A first fan 34 is mounted inside the first adsorption column body 31 and a second fan 36 is mounted inside the second adsorption column body 32.

As illustrated in FIG. 3, as the first fan 34 and the second fan 36 that have the same shape, for example, a sirocco fan is used and the first and second fans 34 and 36 are positioned to face each other, but the first and second fans 34 and 36 are fixed such that the first and second fans 34 and 36 are mounted in vertically opposite directions to each other and face each other.

As illustrated in FIG. 3, the cooling radiator 13 is placed below the first fan 34 and the second fan 36, between the first adsorption column body 31 and the second adsorption column body 32. A power control circuit 39 is placed on the bottom 2B.

FIG. 4 is a diagram illustrating a structure example of the compressor 10 and the compressor 10 includes a first pump unit 51 and a second pump unit 52. The first pump unit 51 includes a cylindrical sleeve 11, a piston 11P placed in the sleeve 11, a head cover 11H, a con rod 11C, and a case section 11F. Similarly, the second pump unit 52 includes a cylindrical sleeve 12, a piston 12P placed in the sleeve 12, a head cover 12H, a con rod 12C, and a case section 12F.

As illustrated in FIG. 4, the sleeves 11 and 12 are also called piston cylinders. A driving motor 53, which is for example, a synchronous motor, has an output shaft 54. Con rods 11C and 12C are rotatably supported on both ends of the output shaft 54.
As illustrated in FIG. 4, the noise buffer (silencer) 38 also serving as the intake filter is placed among the conduit 37, and the first connection conduit 40 and the second connection conduit 41. An end 37B of the conduit 37 is connected to a suction side end 38A of the noise buffer 38 also serving as the intake filter, and a first end 40A of the first connection conduit 40 and a first end 41A of the second connection conduit 41 are connected to a discharge side end 38B of the noise buffer 38 also serving as the intake filter. A second end 40B of the first connection conduit 40 is connected to a suction port 11P of the case section 11F and a second end 41B of the second connection conduit 41 is connected to a suction port 12P of the case section 12F.
An introduction path of the raw air between the noise buffer 38 also serving as the intake filter and the compressor 10 is divided into a plurality of paths, and the first connection conduit 40 and the second connection conduit 41 are connected in parallel between the noise buffer 38 also serving as the intake filter and the compressor 10. In other words, the first connection conduit 40 and the second connection conduit 41 directly connect the suction ports 11P and 12P of the noise buffer 38 also serving as the intake filter and the compressor 10.
As a result, as the raw air introduced from the conduit 37 into the noise buffer 38 also serving as the intake filter passes through the noise buffer 38 also serving as the intake filter, dust is removed by the intake filter, and after noise is reduced, the raw air flows dividedly into the first connection conduit 40 and the second connection conduit 41 and may be introduced into the case section 11F through the suction port 11P of the case section 11F and further, may be introduced into the case section 12F through the suction port 12P of the case section 12F.
The head covers 11H and 12H are commonly connected to the conduit 15 and the generated compressed air is sent through the conduit 15. A heat-dissipating radiator 13 is placed at midstream of the conduit 15.

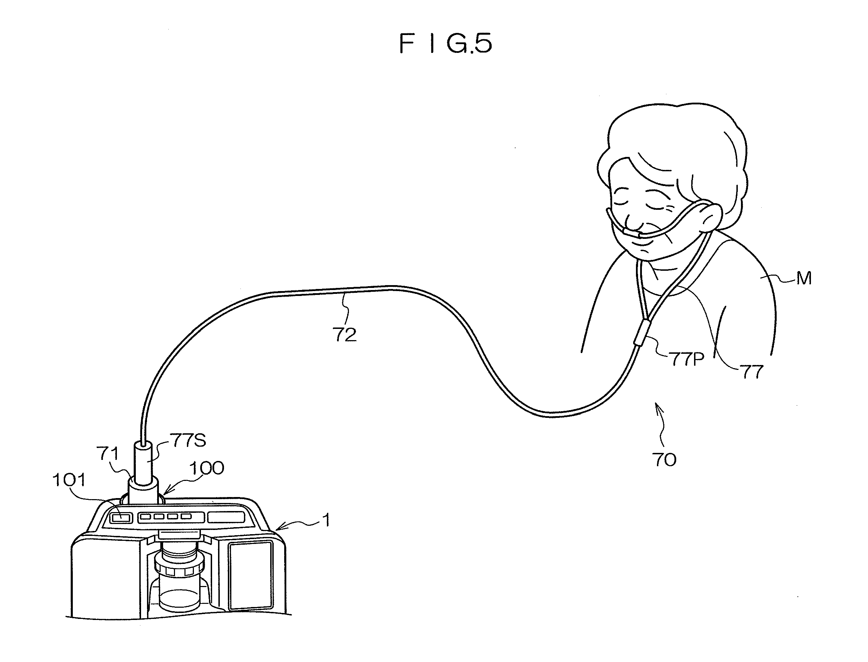

Subsequently, a nasal cannula 70 will be described with reference to FIGS. 5 to 8.

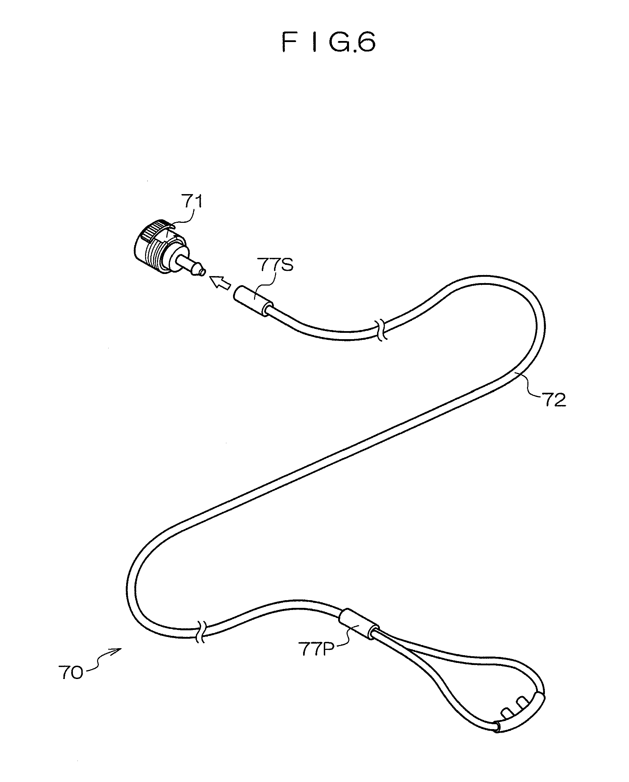

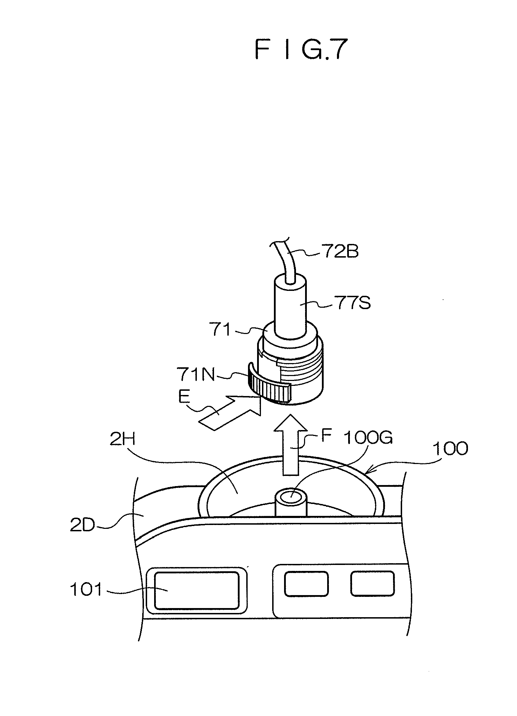

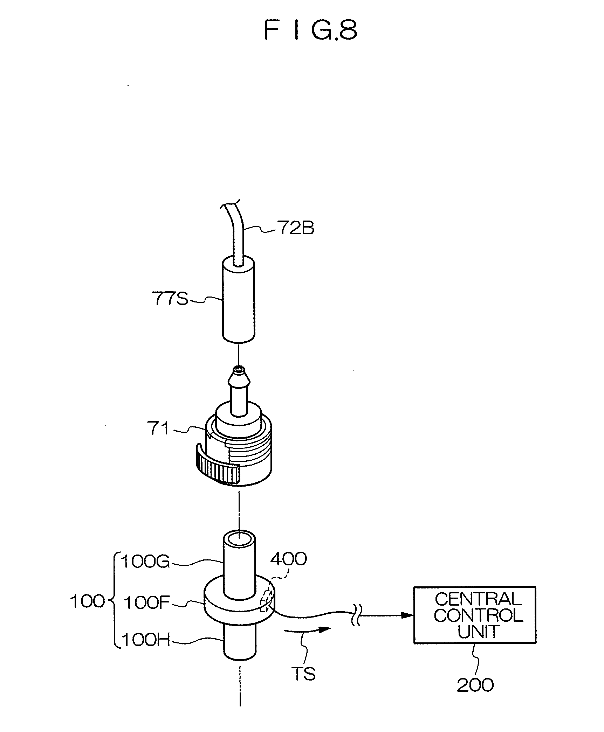

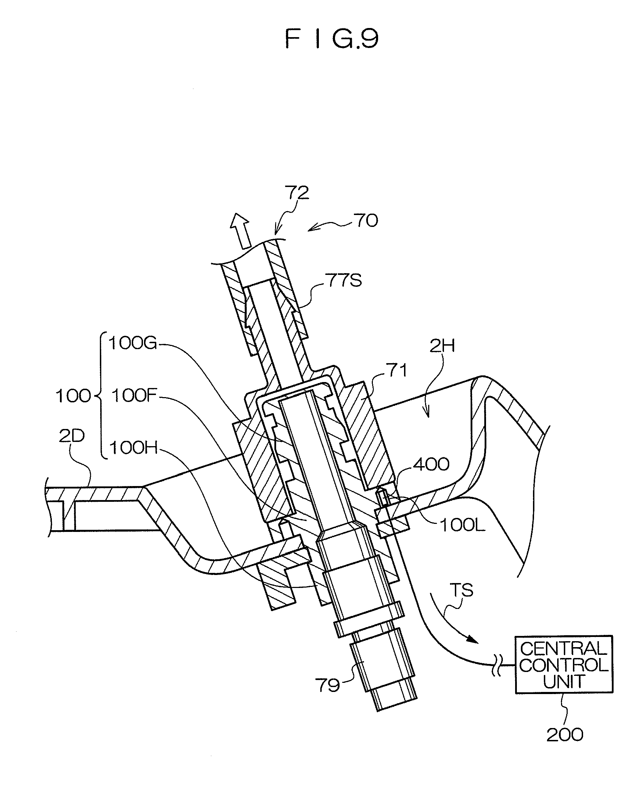

FIG. 5 is a perspective view illustrating that the nasal cannula 70 is connected to an oxygen outlet 100 of an oxygen concentrator 1 by using a coupler socket 71 as one example of a coupler. FIG. 6 is an exploded perspective view illustrating a configuration example of the nasal cannula 70. FIG. 7 is a perspective view illustrating the coupler socket 71 of the nasal cannula 70 and the oxygen outlet 100. FIG. 8 is an exploded perspective view illustrating a connector of a tube of the nasal cannula 70, the coupler socket 71 and the oxygen outlet 100. FIG. 9 is a cross-sectional view illustrating the vicinity of the oxygen outlet 100 and the coupler socket 71.
As illustrated in FIGS. 5 and 6, the nasal cannula 70 is one example of a cannula which a user M wears with his/her ear and nose and includes a mounting portion 77, a tube 72, and the coupler socket 71 as one example of the coupler. The mounting portion 77 is connected to one end of the tube 72 through a connector 77P and the coupler socket 71 is connected to the other end of the tube 72 through a connector 77S.
As illustrated in FIGS. 7 and 8, the coupler socket 71 has a pressing button 71N. As illustrated in FIG. 7, the user M takes out the coupler socket 71 in an F direction (upward) while pressing the pressing button 71N in an E direction to separate the coupler socket 71 from the oxygen outlet 100 by one touch. The user M presses the coupler socket 71 in a reverse direction to the F direction while pressing the pressing button 71N in the E direction to mount and connect the coupler socket 71 on the oxygen outlet 100 by one touch as illustrated in FIG. 9.
The tube 72 illustrated in FIG. 9 is a general connection tube or an extension tube for extending the connection tube.

The coupler socket 71 uses a flexible flame retardant resin, for example, V-0 rank product of US UL-94 standard or a flame retardant resin having performance associated with an oxygen index of 26 or more. The coupler socket 71 is formed by a flame retardant resin having a self-extinguishing property, but the flame retardant resin having the self-extinguishing property is a resin having resistance to flame and when the flame retardant resin contacts flame, the flame retardant resin is ignited, but flame is not propagated and after the flame is removed, the flame retardant resin is self-extinguished within a predetermined time.
As illustrated in FIG. 8, the oxygen outlet 100 includes a ring-shaped flange portion 100F and cylindrical portions 100G and 100H. The flange portion 100f is formed between the cylindrical portion 100G and the cylindrical portion 100H.
The oxygen outlet 100 is made of a metallic material having high thermal conductivity which is not easily rusted, for example, a copper alloy or an aluminum alloy. By forming the oxygen outlet 100 from the metallic material, the oxygen outlet 100 may be able to rapidly transfer heat from the coupler socket 71 to a temperature sensor 400, and as a result, a temperature sensing speed by the temperature sensor 400 may be increased.

As illustrated in FIG. 9, the oxygen outlet 100 is built in for example, a hole portion 100L of the flange portion 100F so that the temperature sensor 400 is not exposed to the outside. As the temperature sensor 400, a thermistor which is cheap and relatively highly sensitive may be preferably adopted, but a thermocouple may be adopted. The temperature sensor 400 is preferably built in the flange portion 100F not to be exposed to the outside because it may be prevented that an attachment such as a scale, and the like is attached to the temperature sensor 400, which cannot accurately sense a temperature and it may be prevented that moisture is attached to the temperature sensor 400, which cannot accurately sense the temperature, even when using a humidifier G illustrated in FIG. 1. Further, the reason is that error detection may be prevented, which is accompanied with the temperature rise by direct heat radiation by a heating mechanism in the winter, a cold region, and the like. The temperature sensor 400 may be fixed into, for example, the hole portion 100L of the flange portion 100F by using an adhesive agent, but may be fixed by screw fixation.

As illustrated in FIG. 9, a joint 79 is connected to the cylindrical portion 100H of the oxygen outlet 100 formed at a concave portion 2H in the rear of a display unit 128 on a top 2D of the oxygen concentrator 1. The joint 79 is connected to supply concentrated oxygen to the tube 72 side through the oxygen outlet 100. The temperature sensor 400 is electrically connected to a central control unit 200 and the temperature sensor 400 supplies the central control unit 200 with temperature information TS of the oxygen outlet 100 sensed by the temperature sensor 400. The central control unit 200 controls various operations for separating the concentrated oxygen. Since the temperature sensor 400 is provided at the concave portion 2H in the rear of the oxygen concentrator 1, error detection may be prevented, which is accompanied with the temperature rise by direct heat radiation by the heating mechanism in the winter, the cold region, and the like.
As illustrated in FIGS. 5 and 9, the coupler socket 71 of the nasal cannula 70 is removably connected to the oxygen outlet 100. The coupler socket 71 is connected to the nasal cannula 70 through the tube 72. The user may inhale, for example, oxygen having a flow corresponding to a maximum flow of 5 L/min. and concentrated at approximately 90% or more, through the nasal cannula 70, the tube 72, and the oxygen outlet 100.

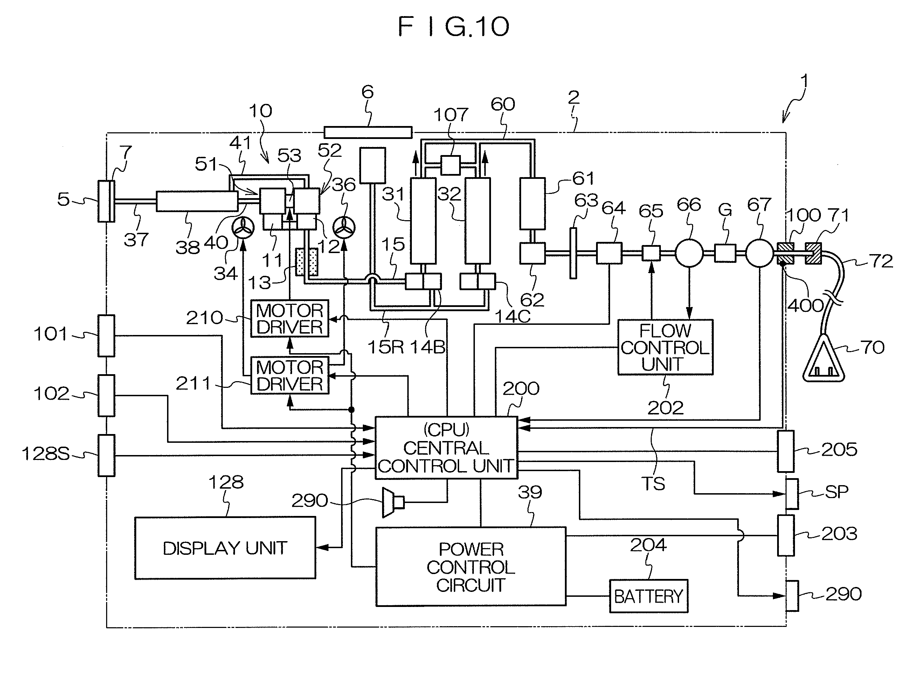

Herein, referring to FIG. 10, a system configuration example of the oxygen concentrator 1 as described above will be described.
FIG. 10 is a diagram illustrating the system configuration example of the oxygen concentrator 1.
A double line illustrated in FIG. 10 represents a conduit serving as a path for the raw air, oxygen gas, and nitrogen gas. A thin solid line represents a wire for power supplying or an electrical signal. The main case 2 of the oxygen concentrator 1 illustrated in FIG. 10 is represented by a dashed line and the main case 2 is an airtight container that hermetically seals components placed therein.

As illustrated in FIG. 10, the main case 2 includes the air introduction port 5 for introducing raw air as outdoor air, the air introduction port filter 7 and the exhaust port 6 for exhausting raw air. The air introduction port filter 7 for removing impurities such as dust, and the like in the air is replaceably placed at the air introduction port 5. When the compressor 10 operates, the raw air is introduced into the compressor 10 through the internal conduit 37, the noise buffer 38 also serving as the intake filter, and the first connection conduit 40 and the second connection conduit 41 connected to the noise buffer 38 also serving as the intake filter in parallel via the air introduction port filter 7.

As such, the raw air is introduced into the compressor 10 to become the compressed air, but heat is generated when the raw air is compressed. As a result, the compressor 10, in particular, the sleeves 11 and 12 are cooled by blowing from the first fan 34 and the second fan 36 for cooling. The compressed air sent from the compressor 10 through the conduit 15 is cooled by the radiator 13.
By cooling the compressed air, the temperature of zeolite as an adsorbent of which a function deteriorates at high temperature may be prevented from being increased. As a result, zeolite may sufficiently serve as the adsorbent for separating oxygen by adsorption of nitrogen and oxygen may be concentrated up to approximately 90% or more.

The first adsorption column body 31 and the second adsorption column body 32 as examples of adsorption members placed in line are placed in parallel vertically. The 3-way switching valves 14B and 14C are connected to the first adsorption column body 31 and the second adsorption column body 32, respectively. One end of one 3-way switching valve 14B is connected to the conduit 15. One 3-way switching valve 14B and the other 3-way switching valve 14C are connected to each other and one end of the other 3-way switching valve 14C is connected to a conduit 15R. An end of the conduit 15R reaches the exhaust port 6.
The 3-way switching valves 14B and 14C are connected to correspond to the first adsorption column body 31 and the second adsorption column body 32, respectively. The compressed air generated from the compressor 10 are alternately supplied to the first adsorption column body 31 and the second adsorption column body 32 through the conduit 15, and the 3-way switching valves 14B and 14C.

Zeolite as a catalyst adsorbent is stored in each of the first adsorption column body 31 and the second adsorption column body 32. The zeolite is X-type zeolite in which for example, a ratio of Si₂O₃/Al₂O₃ is in the range of 2.0 to 3.0, and zeolite in which at least 88% of a tetrahedral unit of Al₂O₃ is combined with lithium cation is used to increase an adsorption amount of nitrogen per unit weight. The zeolite has particularly, a granule measurement value which is less than 1 mm and at least 88% of the tetrahedral unit is preferably fused with lithium cation. By using zeolite, the amount of used raw air required to separate oxygen may be reduced as compared with a case of using other adsorbent. As a result, the compressor 10 for generating the compressed air may be further miniaturized and low noise of the compressor 10 may be achieved.

As illustrated in FIG. 10, a uniform-pressure valve 107 constituted by a check valve, a diaphragm valve, and an opening/closing valve is connected to outlets of the first adsorption column body 31 and the second adsorption column body 32. A joined conduit 60 is connected to a downstream side of the uniform-pressure valve 107 and a buffer 61 is connected to the conduit 60. The buffer 61 is an oxygen storing container for storing oxygen having a concentration of approximately 90% or more which is separately generated from the first adsorption column body 31 and the second adsorption column body 32.

As illustrated in FIG. 10, a pressure adjuster 62 is connected to a downstream side of the buffer 61 and the pressure adjuster 62 is a regulator that automatically adjusts the pressure of oxygen at an outlet of the buffer 61 to be uniform. A zirconia or ultrasonic oxygen concentration sensor 64 is connected to a downstream side of the pressure adjuster 62 through a filter 63 and the oxygen concentration sensor 64 detects the concentration of oxygen intermittently (every 10 to 30 minutes) or continuously.

As illustrated in FIG. 10, a proportional opening rate valve 65 is connected to the buffer 61. The proportional opening rate valve 65 is opened/closed in link with setting button operation of the oxygen flow setting button 102 by a signal from a flow control unit 202 according to a command of a central control unit 200. An oxygen flow sensor 66 is connected to the proportional opening rate valve 65. The humidifier G and an oxygen flow sensor 67 are connected to the oxygen flow sensor 66. The oxygen outlet 100 is connected to a stage subsequent to the oxygen flow sensor 67.
A coupler socket 71 of a nasal cannula 70 is removably connected to the oxygen outlet 100 in FIG. 10. The coupler socket 71 is connected to the nasal cannula 70 through a tube 72. A patient may inhale for example, oxygen having a flow corresponding to a maximum flow of 5 L/min. and concentrated at approximately 90% or more, through the nasal cannula 70.
A temperature sensor 400 embedded in the oxygen outlet 100 is electrically connected to a central control unit 200 and temperature information TS of the oxygen outlet 100 sensed by the temperature sensor 400 is provided to the central control unit 200.

Subsequently, a power system will be described with reference to FIG. 10.
A connector 203 of an AC (utility AC) power supply illustrated in FIG. 10 is electrically connected to the power control circuit 39 and the power control circuit 39 rectifies AC voltage of the utility AC power supply into predetermined DC voltage. A built-in battery 204 is built in the main case 2. The built-in battery 204 is a secondary battery which is repeatedly rechargeable and the built-in battery 204 may be recharged by receiving power supplied from the power control circuit 39.

As a result, the central control unit 200 of FIG. 1 controls the power control circuit 39, such that the power control circuit 39 may be, for example, used while being automatically switched to any one supply state of a first power supply state in which the power control circuit 39 operates by receiving power supplied from an AC adapter 203 and a second power supply state in which the power control circuit 39 operates by receiving power supplied from the built-in battery 204. As the built-in battery 204, a lithium ion secondary battery and a lithium hydrogen ion secondary battery, which are low in memory effect while charging and are fully charged even while recharging, may be used, but a nickel cadmium battery or a nickel hydrogen battery in the related art may be used.

The central control unit 200 of FIG. 10 is electrically connected to a motor driver 210 and a fan motor driver 211. The central control unit 200 stores a program to switch an operation mode to an optimal operation mode depending on the amount of separated oxygen. The motor driver 210 and the fan motor driver 211 control to automatically drive the compressor 10, and the first fan 34 and the second fan 36 at a high speed when a large amount of oxygen is separated and to rotatably drive the compressor 10, and the first fan 34 and the second fan 36 at a low speed when a small amount of oxygen is separated, according to the command of the central control unit 200.

A read only memory (ROM) storing a predetermined operation program is built in the central control unit 200 and a circuit constituted by an external storage device, a volatile memory, a temporary storage device, and a real-time clock is electrically connected to the central control unit 200. The central control unit 200 is accessible by connecting with an external communication line, and the like through a communication connector 205.
By on/off-controlling the 3-way switching valves 14B and 14C and the uniform-pressure valve 107 illustrated in FIG. 10, a control circuit (not illustrated) that controls unnecessary gas in the first adsorption column body 31 and the second adsorption column body 32 to be desorbed, the pressure adjuster 62, the flow control unit 202, and the oxygen concentration sensor 64 are electrically connected to the central control unit 200. The flow control unit 202 controls the proportional opening rate valve 65, and oxygen flow values of the oxygen flow sensor 66 and the oxygen flow sensor 67 are sent to the central control unit 200. The oxygen flow setting button 102, the display unit 128, the power switch 101, and a display switch 128S are electrically connected to the central control unit 200 illustrated in FIG. 10.
The central control unit 200 is electrically connected to a warning means SP such as a buzzer or a lamp. The warning means SP may warn a user of interruption of supply of oxygen to the user through sound or light when the supply of oxygen is interrupted. A speaker 290 is electrically connected to the central control unit 200 and the speaker 290 guides interruption of the supply of oxygen to the user by voice when the supply of oxygen is interrupted. Interruption of the supply of oxygen to the user is displayed on the display unit 128 by texts or drawings when the supply of oxygen is interrupted.

The oxygen flow setting button 102 may set the flow of oxygen whenever for example, operating oxygen concentrated at approximately 90% or more from 0.25 L (liter) to the maximum 5 L by 0.25 L per minute. As the display unit 128, for example, a display device such as a liquid crystal monitor displaying 7 segments, and the like is used. For example, display items including the oxygen flow, an oxygen lamp, warning icons (tube bending, separation of the humidifier, decrease in oxygen concentration, stoppage of power supplying, a residual quantity of the battery, battery in operation, and a charging lamp), an accumulation time, and the like may be displayed in the display unit 128.

The compressor 10 illustrated in FIG. 10 generates only the compressed air to send the compressed air to the first adsorption column body 31 and the second adsorption column body 32 by static pressure swing adsorption (PSA) and adsorbs nitrogen in the compressed air by the adsorbent in the first adsorption column body 31 and the second adsorption column body 32, as already described. Although the driving motor 53 of the compressor 10 is the synchronous motor, the driving motor 53 may be other motors, for example, a single phase AC induction motor or a single phase 4-pole AC synchronous motor and is not particularly limited to a specific type.

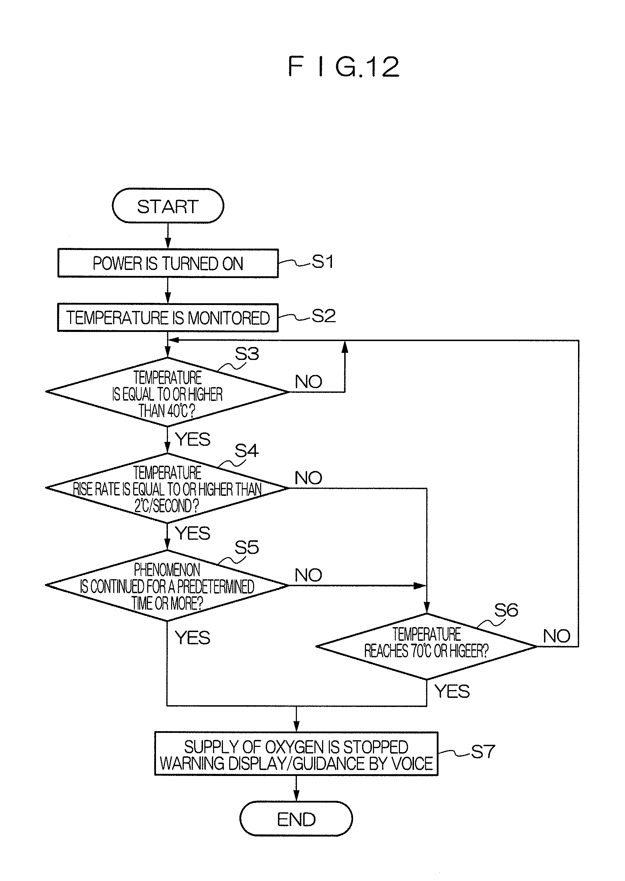

Subsequently, an operation example of the aforementioned oxygen concentrator 1 will be described with reference to FIGS. 10 to 12.
As illustrated in FIGS. 5 and 9, when a user M inhales the concentrated oxygen by using the cannula 70 with respect to the oxygen concentrator 1, the user M pushes the coupler socket 71 at the front end of the tube 72 into the oxygen outlet 100 to connect the coupler socket and the oxygen outlet 100.
In step S1 of FIG. 12, when the user M turns on the power switch 101 illustrated in FIG. 10 to start inhaling the concentrated oxygen, in step S2, the temperature information TS (output signal) is received from the temperature sensor 400 to start monitoring the temperature at the oxygen outlet 100.
The central control unit 200 illustrated in FIG. 10 gives a command to the motor driver 210 to allow the motor driver 210 to start the driving motor 53 of the compressor 10, thus consecutively rotating the output shaft 54 of the driving motor 53 illustrated in FIG. 4. As a result, a piston 11P of a first head section 51 and a piston 12P of a second head section 52 illustrated in FIG. 4 move reciprocatively.

When the compressor 10 operates, the raw air is introduced from the air introduction port 5 illustrated in FIG. 10 and the impurities such as dust are removed by the filter 7. Thereafter, the raw air is introduced into the sleeves 11 and 12 via the suction ports 11P and 12P of the compressor 10 in FIG. 4, through the internal conduit 37, the noise buffer 38 also serving as the intake filter, and the first connection conduit 40 and the second connection conduit 41 connected in parallel. As such, as the raw air introduced from the conduit 37 illustrated in FIG. 4 into the noise buffer 38 also serving as the intake filter passes through the noise buffer 38 also serving as the intake filter, dust and the like is removed, and after noise is reduced, the raw air flows dividedly into the first connection conduit 40 and the second connection conduit 41 connected in parallel and may be introduced into the case section 11F through the suction port 11P of the case section 11F and further, may be introduced into the case section 12F through the suction port 12P of the case section 12F. When the piston 11P and the piston 12P of FIG. 4 are positioned at top dead points, the raw air in the sleeve 11 and the sleeve 12 is compressed. On the contrary, when the piston 11P and the piston 12P are positioned at bottom dead points, the raw air is suctioned into the sleeve 11 and the sleeve 12.
The first connection conduit 40 and the second connection conduit 41 divide an introduction path of the raw air between the noise buffer 38 also serving as the intake filter and the compressor 10 into a plurality of systems to be parallel and directly connect the noise buffer 38 also serving as the intake filter and the suction ports 11P and 12P of the compressor 10 to each other. As a result, the amount of raw air which should be sent per one of the first connection conduit 40 and the second connection conduit 41 may be reduced. In other words, although the diameters of the first conduit 40 and the second conduit 41 are set to be small, the pressure loss is not increased. Since noise per conduit of the first connection conduit 40 and the second connection conduit 41 at the time of sending the raw air to the compressor 10 is also remarkably decreased, noise may be reduced even though the first connection conduit 40 and the second connection conduit 41 are used as compared with the case of forming the branch conduits by branching the midstream of one conduit in the related art.
The compressed air generated by the compressor 10 illustrated in FIG. 10 may be supplied to the first adsorption column body 13 and the second adsorption column body 32 through the conduit 15.

Meanwhile, the central control unit 200 illustrated in FIG. 10 gives a command to the motor driver 211 to rotate the first fan 34 and the second fan 36. When the compressor 10 compresses the raw air to generate the compressed air, the sleeves 11 and 12 of the compressor 10 are cooled by blowing from the first fan 34 and the second fan 36, respectively and the compressed air that passes through the conduit 15 is cooled by passing through the radiator 13. The compressed air adsorbs nitrogen by passing through the adsorbent in the first adsorption column body 31 and the second adsorption column body 32 through the conduit 15 and the 3-way switching valves 14B and 14C, such that oxygen is separated and generated from the compressed air. The buffer 61 may store oxygen having a concentration of approximately 90% or more which is separated and generated.

The oxygen concentration sensor 66 of FIG. 10 detects the concentration of oxygen from the buffer 61. The proportional opening rate valve 65 is opened/closed in link with the oxygen flow setting button 102. Oxygen is supplied to the nasal cannula 70 through the oxygen outlet 100. As a result, the patient may inhale oxygen concentrated at approximately 90% or more at the maximum flow of, for example, 5 L/min through the nasal cannula 70.

However, as described above, when the oxygen concentrator is exposed to fire or an abnormal high-temperature environment at the time of user inhaling the concentrated oxygen by using the nasal cannula 70, the tube 72 of the nasal cannula 70 may be directly ignited or the surface of the oxygen concentrator 1 may be in a high-temperature state.
Therefore, for example, when the tube 72 of the nasal cannula 70 is directly ignited with flame of a cigarette, an operation of interrupting the supply of the concentrated oxygen for ensuring safety will be described. Apart from the direct ignition, when the surface of the oxygen concentrator 1 becomes the high-temperature state, an operation of interrupting the supply of the concentrated oxygen for ensuring safety will be described.

First, the user M illustrated in FIG. 5, for example, smokes and the tube 72 of the nasal cannula 70 is ignited with the flame of the cigarette, the flame is propagated toward the coupler socket 71 through the tube 72 and the flame reaches to ignite the coupler socket 71, which is combusted or overheated by oxygen in the air. When the coupler socket 71 is combusted or overheated, heat is transferred from the coupler socket 71 to the oxygen outlet 100, and as a result, the temperature of the oxygen outlet 100 rises.
Therefore, in step S3 of FIG. 12, the temperature sensor 400 measures the temperature of the oxygen outlet 100 and sends the temperature information TS to the central control unit 200 illustrated in FIG. 10. The control unit 200 judges whether the measured temperature of the oxygen outlet 100 is equal to or higher than a predetermined temperature - a temperature equal to or higher than a predetermined use environment temperature - for example, at 40°C or higher.

In step S3, when the measured temperature of the oxygen outlet 100 is equal to or higher than 40°C which is the predetermined temperature and further, in step S4, when the central control unit 200 judges that a phenomenon in which the measured temperature of the oxygen outlet 100 is equal to or higher than a temperature rise rate of 2°C/second (2°C rises in 1 second) which is a rise value of the temperature per predetermined unit time is continued for a predetermined continued time or more, for example, 3 seconds or more in step S5, in step S7, the central control unit 200 of FIG. 10 gives a command to a motor driver 210 and stops a generation operation of compressed air by a pump 10.
As a result, even when the cannula 70 or the tube 72 is directly ignited, such that the flame reaches the coupler socket 71 and thus the temperature of the oxygen outlet 100 rises, the supply of the concentrated oxygen to the oxygen outlet 100 and the nasal cannula 70 may be interrupted for ensuring safety.

As illustrated in step S7 of FIG. 12, with the interruption of the concentrated oxygen, the central control unit 200 of FIG. 10 gives a warning to the user by using sound or light, or both through a warning means SP and a speaker 290 guides interruption of the supply of oxygen to the user by voice. Further, interruption of the supply of oxygen to the user is displayed on the display unit 128 by texts or drawings. As a result, the user may certainly recognize interruption of the supply of the concentrated oxygen by means of the ears and the eyes.
Since the coupler socket 71 is made of the flame retardant resin having the self-extinguishing property as described above, when the supply of the concentrated oxygen from the oxygen outlet 100 stops, combustion of the coupler socket 71 is not continued but self-extinguished.
When the nasal cannula 70 is made of a fluorine resin, the nasal cannula 70 may be self-extinguished although the nasal cannula 70 is ignited, but since the fluorine resin is a hard material, flexibility is short and it is difficult for the user to mount and fit the nasal cannula 70.

In step S4 of FIG. 12, when the temperature rise rate is less than a predetermined temperature rise rate or a state in which the temperature rise rate is equal to or higher than the predetermined temperature rise rate in step S5 is not continued for a predetermined continued time or more, the oxygen concentrator 1 itself illustrated in FIG. 1 is exposed to the fire or the abnormal high-temperature environment, such that the surface of the oxygen concentrator 1 becomes the high-temperature state. Therefore, the process proceeds to step S6.
In step S6, when the temperature sensor 400 of the oxygen outlet 100 senses that the temperature of the oxygen outlet 100 reaches a predetermined different temperature, for example, 70°C or higher, the central control unit 200 of FIG. 10 gives a command to the motor driver 21 to stop an operation of a motor 53 of a compressor 10, thereby interrupting the supply of the concentrated oxygen. By step 6, error detection may be prevented, which is accompanied with the temperature rise by direct heat radiation by the heating mechanism in the winter, the cold region, and the like.

As a result, a main case 2 of the oxygen concentrator 1 may be prevented from being combusted in a spreading fire while a simple structure is adopted. As illustrated in step S7 of FIG. 12, with the interruption of the concentrated oxygen, the central control unit 200 of FIG. 10 gives the warning to the user by using the sound or light, or both through the warning means SP and the speaker 290 guides interruption of the supply of oxygen to the user by the voice. Further, interruption of the supply of oxygen to the user is displayed on the display unit 128 by the texts or drawings. As a result, the user may certainly recognize interruption of the supply of the concentrated oxygen by means of the ears and the eyes.
When the supply of oxygen stops, the combusted coupler socket 71 is extinguished, and as a result, fire spreading to the oxygen concentrator 1 may be prevented and a time until the coupler socket 71 is extinguished after the coupler socket 71 is ignited is in the range of approximately 15 to 30 seconds.

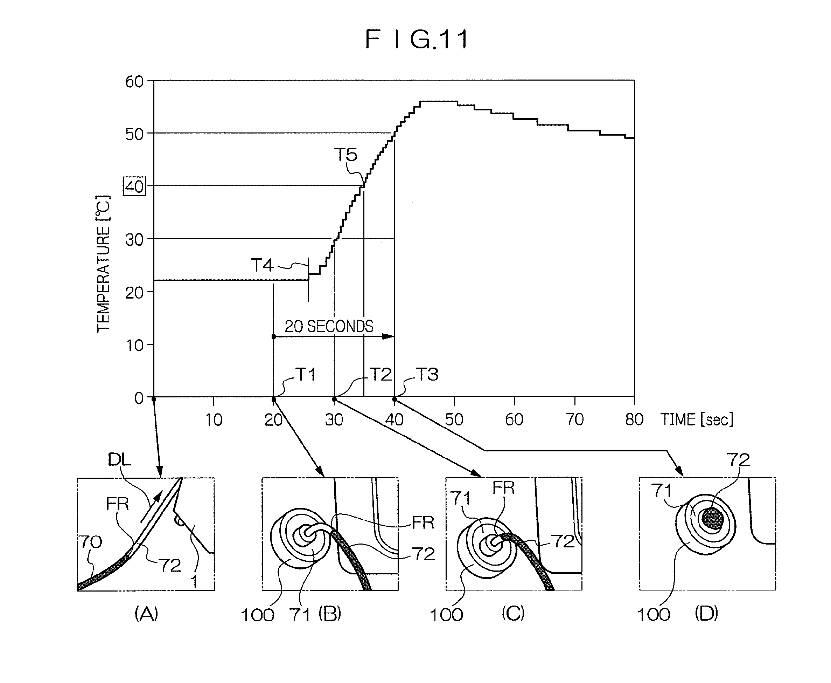

Herein, referring to FIG. 11, an example of a temperature change in the oxygen outlet 100 until the concentrated oxygen is stopped and flame is extinguished after the tube 72 of the nasal cannula 70 is ignited will be described simply.
FIG. 11 illustrates an example of a temperature change in the oxygen outlet 100 until the concentrated oxygen is stopped and flame is extinguished after the tube 72 of the nasal cannula 70 is ignited. The temperature change in the oxygen outlet 100 is sent from the temperature sensor 400 illustrated in FIG. 10 to the central control unit 200 as the temperature information TS.
FIG. 11A illustrates a state in which the tube 72 of the nasal cannula 70 is ignited and flame FR in the tube 72 is thus propagated in a DL direction and in this case, the temperature of the oxygen outlet 100 is 22°C as a room temperature. FIG. 11B illustrates a state just before the flame FR propagated into the tube 72 reaches the coupler socket 71. The temperature of the oxygen outlet 100 in this case is still the room temperature.

FIG. 11C illustrates that the flame FR reaches the coupler socket 71 to combust the coupler socket 71 and heat generated by combustion of the coupler socket 71 is transferred to the oxygen outlet 100 through the oxygen outlet 100 having excellent thermal conductivity, and as a result, the temperature sensor 400 illustrated in FIG. 10 measures the rise in temperature of the oxygen outlet 100. As such, when the flame FR is close to the coupler socket 71, the temperature of the oxygen outlet 100 starts slowly rising from 22°C at a time T4.
FIG. 11D illustrates a time when the temperature of the oxygen outlet 100 reaches, for example, 50°C at a time T3 when 20 seconds elapsed from a time T1 of FIG. 11B. When 10 seconds elapsed from the time T2 to the time T3, the temperature of the oxygen outlet 100 rises by 20°C.
Between a time T5 and the time T3, the measured temperature of the oxygen outlet 100 is 40°C or higher, and between the time T5 and 5 seconds after the time T3, the temperature rises at the predetermined temperature rise rate of 2°C/1 second and the temperature rise continues for 5 seconds which is 3 seconds or more.

As a result, the central control unit 200 of FIG. 11 gives a command to the motor driver 21 to stop the operation of the motor 53 of the compressor 10, thereby stopping the supply of the concentrated oxygen. As a result, since the concentrated oxygen is not discharged from the oxygen outlet 100, the flame of the coupler socket 71 is settled and extinguished.
As such, the main case 2 of the oxygen concentrator 1 may be prevented from being combusted in a spreading fire while the simple structure is adopted. With the interruption of the concentrated oxygen, the central control unit 200 of FIG. 10 gives the warning to the user by using the sound or light, or both through the warning means SP and the speaker 290 guides interruption of the supply of oxygen to the user by the voice. Further, interruption of the supply of oxygen to the user is displayed on the display unit 128 by the texts or drawings. As a result, the user may certainly recognize interruption of the supply of the concentrated oxygen by means of the ears and the eyes.

In the oxygen concentrator according to the embodiment of the present invention, safety may be ensured by interrupting oxygen when the oxygen concentrator is exposed to the fire or abnormal high-temperature environment at the time of user inhaling oxygen with the cannula. When the oxygen concentrator is exposed under the abnormal high-temperature environment such as the fire, and the like, fire spreading to the oxygen concentrator or the fire of the device itself may be prevented.
The control unit stops the operation of the compressor to interrupt the supply of oxygen when the temperature sensed by the temperature sensor reaches the predetermined temperature or higher and further, a continued time when the temperature rise rate which is the temperature rise value per unit time reaches a predetermined value or more is continued for a predetermined continued time or more. As a result, the control unit senses the rise in temperature of the oxygen outlet to thereby certainly interrupt the supply of oxygen.

In the case where the temperature sensed by the temperature sensor reaches the predetermined temperature or higher and further, the temperature rise rate is less than the predetermined value, the control unit stops the operation of the compressor to interrupt the supply of oxygen when the temperature sensed by the temperature sensor reaches an additionally set temperature or higher over the predetermined temperature. As a result, the control unit may sense the rise in temperature of the oxygen outlet to thereby certainly interrupt the supply of oxygen and prevent a main body of the oxygen concentrator from being combusted in a spreading fire.
The temperature sensor is the thermistor and the temperature sensor is built in the oxygen outlet. As a result, since cost-down is achieved by using the thermistor which is cheap as the temperature sensor and further, the temperature sensor is built in the oxygen outlet, the temperature may be prevented from not being accurately sensed due to the attachment such as the scale, and the like attached to the temperature sensor and the temperature may be prevented from not being accurately sensed due to moisture attached to the temperature sensor even when using the humidifier.

Since the coupler is made of the flame retardant resin material, when the supply of oxygen from the oxygen outlet is stopped, the flame of the coupler is removed.
Since the oxygen outlet is made of metal having thermal conductivity, heat by combustion of the coupler may be rapidly transferred to the temperature sensor through the oxygen outlet, and as a result, a temperature sensing time is shortened.
Since the oxygen concentrator includes warning means notifying that oxygen supply from the oxygen outlet is interrupted by the command of the control unit and a speaker notifying that oxygen supply from the oxygen outlet is interrupted by the command of the control unit by the voice, the oxygen concentrator may certainly notify the user of stopping the supply of oxygen.
Further, since static pressure swing adsorption (PSA) by only the compressed air sends only the compressed air to an adsorption column body to adsorb nitrogen, miniaturization of the compressor and simplification of the structure thereof may be achieved as compared with positive and negative pressure swing adsorption (VPSA) by the compressed air and decompressed air.

However, the present invention is not limited to the embodiment and various modifications and changes of the present invention can be made and various transformations can be made within the scope of the appended claims.
The measured temperature of the oxygen outlet 100 is 40°C or higher, but may adopt other values including 45°C or higher, and the like. A predetermined temperature rise rate at the oxygen outlet 100 is 2°C/sec., but may be, for example, 1°C/sec. The temperature rise rate is continued for 3 seconds or longer, but may be continued, for example, 5 seconds or longer or may be set arbitrarily.
The temperature sensor may be embedded in the oxygen outlet, but may be placed outside the oxygen outlet. The oxygen outlet may be made of a metallic material having excellent thermal conductivity, but instead thereof, may be made of, for example, a fire retardant material such as ceramics, which is lower than metal in thermal conductivity.
The driving motor of the illustrated compressor 10 is for example, the 5 L-class_motor, but is not limited thereto and may adopt for example, a motor suitable for 3 L class and the like. The type of the compressor is not particularly limited and may adopt a predetermined type.
The oxygen concentrator is not limited to the oxygen concentrator using the pressure swing adsorption, but may adopt a membrane type oxygen concentrator.

### Reference Signs List

1: Oxygen concentrator
2: Main case
10: Compressor
70: Nasal cannula (one example of cannula)
71: Coupler socket (one example of coupler)
72: Tube
100: Oxygen outlet
290: Speaker
400: Temperature sensor
SP: Warning means

## Claims

1. An oxygen concentrator, comprising:
concentrated oxygen separating means for separating concentrated oxygen from raw air;
oxygen outlet for supplying the concentrated oxygen separated by the concentrated oxygen separating means;
a coupler mounted on a tube of a cannula, and removably connecting the tube to the oxygen outlet, and moreover having a temperature sensor; and
a control unit stopping operation of the concentrated oxygen separating means to interrupt the supply of oxygen when a temperature sensed by the temperature sensor reaches a predetermined temperature or higher.

2. The oxygen concentrator according to claim 1, wherein the control unit stops operation of a compressor, which serves as the concentrated oxygen separating means, to interrupt the supply of oxygen when the temperature sensed by the temperature sensor reaches the predetermined temperature or higher, and when a continued time, during which a temperature rise rate which is the temperature rise value per unit time reaches a predetermined value or more, is continued for a predetermined continued time or longer.

3. The oxygen concentrator according to claim 1 or 2, wherein in the case where the temperature sensed by the temperature sensor reaches the predetermined temperature or higher and when the temperature rise rate is less than the predetermined value, the control unit stops the operation of the compressor to interrupt the supply of oxygen when the temperature sensed by the temperature sensor reaches an additionally set temperature or higher over the predetermined temperature.

4. The oxygen concentrator according to any of claims 1 to 3, wherein the temperature sensor is a thermistor and the temperature sensor is built in the oxygen outlet.

5. The oxygen concentrator according to any of claims 1 to 4, wherein the coupler is made of a flame retardant resin material.

6. The oxygen concentrator of any of claims 1 to 5, wherein the oxygen outlet is made of metal having thermal conductivity.

7. The oxygen concentrator of any of claims 1 to 6, further comprising:
warning means for notifying that oxygen supply from the oxygen outlet is interrupted f by a command of the control unit; and
a speaker notifying that oxygen supply from the oxygen outlet is interrupted by the command of the control unit by voice.
